# EUROPÄISCHE PATENTSCHRIFT

(11) **EP 1 077 377 B1**
(45) Veröffentlichungstag und Bekanntmachung des Hinweises auf die Patenterteilung: **22.02.2006**
(21) Anmeldenummer: 00114266.0
(22) Anmeldetag: 04.07.2000
(51) Int. Cl.: G01N 33/44

(54) **Verfahren und Vorrichtung zum Ermitteln der Qualität der Oberflächenstruktur von Häuten**
Method and apparatus for determining the quality of the surface structure of skins
Méthode et appareil pour déterminer la qualité de la structure superficielle de peaux

(30) Priorität: 17.08.1999 DE 19938989
(43) Veröffentlichungstag der Anmeldung: 21.02.2001
(73) Patentinhaber: Bayerische Motoren Werke Aktiengesellschaft, 80809 München (DE)
(72) Erfinder: Schmitt, Josef, 84130 Dingolfing (DE); Schachtl, Johann, 94574 Wallerfing (DE); Busler, Max, 94428 Eichenhof (DE)

(56) Entgegenhaltungen:
- EP-A- 0 586 795
- US-A- 3 067 609
- DATABASE WPI Section EI, Week 198419 Derwent Publications Ltd., London, GB; Class S02, AN 1984-119425 XP002199565 & SU 1 033 845 A (MOSC LIGHT IND TECH), 7. August 1983 (1983-08-07)

## Beschreibung

Die Erfindung bezieht sich auf ein Verfahren nach dem Oberbegriff des Anspruchs 1 und auf eine Vorrichtung zur Durchführung des Verfahrens nach dem Oberbegriff des Anspruchs 6.

Bestimmte Lederarten (losnarbiges Leder, Bauchleder, Leder mit loser Faserstruktur) neigen bei einer Weiterverarbeitung wie Laminieren oder Hinterschäumen zur verstärkten Faltenbildung und damit zum Ausschuß.

Bis heute wird die Bestimmung der Verarbeitungsqualität von Lederhäuten und die hierbei erwarteten Veränderungen in der Narbenstruktur in der Lederindustrie durch subjektive Beurteilung anhand von Grenzmustem durchgeführt. Die Vorgänge zum Festlegen von Grenzmustem sind sehr aufwendig. Die Beurteilung der Lederproben bleibt jedoch nach wie vor subjektiv. Dies kann in manchen Fällen dazu führen, daß Leder verwendet wird, das während seiner weiteren Verarbeitung zu Ausschuß führt. Unter weiterer Verarbeitung wird insbesondere ein Stanzen, Laminieren, Kaschieren, Hinterschäumen, Prägen oder ähnliche Verfahren verstanden.

Aus der US-A-3 067 609 ist eine Vorrichtung zur Ermittlung der Qualität der Ober flächenstruktur von Lederhäuten bekannt. Hierbei wird ein Probenstreifen der Lederhaut eingespannt und gestreckt, in dem zwei bewegliche untere Aufnahmen vorgesehen sind, die mit einer gemeinsamen Abdeckplattte zusammenwirken, um einen Probenstreifen aus Leder einzuspannen. Gleichzeitig ist an der Abdeckplatte eine Auswölbung vorgesehen, die mit einer Nut, die zur Hälfte in jeweils einer der beiden unteren Aufnahmen vorgesehen ist, zusammenwirkt. Dadurch wird der Lederstreifen in diesem Bereich gekrümmt bzw. gestaucht, und die Anzahl an Falten pro Längeneinheit kann bestimmt werden.

Es ist deshalb wünschenswert, ein objektives Beurteilungsverfahren zu erhalten, damit Ausschuß und Abfall vermieden werden.

Diese Aufgabe wird erfindungsgemäß durch die Merkmale des Anspruchs 1 bzw. des Anspruchs 6 gelöst.

Die Lösung basiert auf der Erkenntnis, daß als Maß der Qualität der Oberflächenstruktur von Häuten deren Rauhtiefe gemessen wird. Hierzu wird ein schmaler Probenstreifen der zu untersuchenden Haut in seiner Axialrichtung zuerst mit einer Vorspannung belastet und dann die Rauhtiefe ermittelt. Anschließend wird die Rauhtiefe an der gekrümmten Oberfläche ermittelt und mit der zuerst erfaßten Rauhtiefe verglichen. Das Verhältnis der ermittelten Rauhtiefen zueinander bildet ein Maß für die Qualität der Oberflächenstruktur. Als Rauhtiefe wird hier der Mittenrauhwert Rₐ verwendet.

Versuchsreihen haben ergeben, daß z.B. Verhältniswerte von unter 100% bei konkaver Form eine Eignung für eine Weiterverarbeitung signalisieren, wobei die Grenzen je nach Weiterverarbeitungsschritt variieren können und zuvor durch Testreihen ermittelt werden müssen.

Unter dem Begriff Haut werden vorliegend Lederhäute, aber auch Sprühhäute wie PUR Häute, Kunstleder und Folien verstanden.

Die Weiterbildung nach Anspruch 4 berücksichtigt die Tatsache, daß konvex gekrümmte Oberflächen immer unter einer Vorspannung aufgrund der Weiterverarbeitung stehen.

Gemäß Anspruch 5 kann die Rauhtiefe mit Hilfe von optischen Verfahren, z.B. topometrische 3D-Sensoren oder durch sonstige Taster ermittelt werden. Derartige Geräte sind auf dem Markt frei verfügbar.

Ein weiterer Vorteil der Erfindung besteht darin, daß es möglich ist, die im Produktionsprozeß auftretenden Dehnungskräfte auszuüben und die sich hierbei einstellende Dehnung zu messen. Damit erhält man objektive Aussagen über das Dehnverhalten der verwendeten Haut.

Mit dem erfindungsgemäßen Verfahren ist es somit möglich, Kenndaten für die Verarbeitung von Häuten, insbesondere Lederhäuten, zu ermitteln. Damit lassen sich zuverlässig Machbarkeitsanalysen im Vorfeld bei der Entwicklung neuer Materialien und neuer Verarbeitungsverfahren erstellen. Weiterhin kann eine Überwachung der Prozeßschritte beim Lederhersteller, z.B. beim Gerben, Zurichten, Weichmachen durch Millen, Walken, Stollen, etc. erfolgen und Prozesse, die sich negativ auf die beabsichtigte weitere Verarbeitung der Häute auswirkt, eliminiert oder verbessert werden. Weiterhin werden dadurch unnötiger Abfall und die Reduzierung von Ausschuß erreicht. Auch können eventuelle Rücksendungen nicht geeigneter Lederhäute rechtzeitig vor Beginn einer Weiterverarbeitung veranlaßt werden. Ein weiterer Vorteil besteht darin, die Häute entsprechend ihrer tatsächlichen Qualität gezielt dem jeweiligen unterschiedlichen Weiterverarbeitungsprozessen zu zuführen, da nicht jeder Weiterverarbeitungsprozeß bzw. jedes Produkt gleiche Materialqualität erfordert.

Anspruch 6 beschreibt eine Vorrichtung zur Durchführung des Verfahrens. Mit dieser Vorrichtung kann auch zuverlässig das Dehnungsverhalten ermittelt werden, wobei bevorzugt die Dehnung unter den Belastungen während des Umformvorganges erfolgt und nicht - im Gegensatz zur einschlägigen DIN-Norm - bei einer Kraft, die normiert oberhalb der bei weiteren Formgebungsverfahren auftretenden maximalen Dehnungskräfte liegt.

Die Ansprüche 7 bis 16 beschreiben bevorzugte Ausführungsformen der erfindungsgemäßen Vorrichtung.

Im folgenden wird die Erfindung anhand eines bevorzugten Ausführungsbeispiels näher beschrieben. Es stellen dar:
- Figur 1: einen schematisierten Querschnitt durch eine erfindungsgemäße Vorrichtung mit einem eingespannten Probenstreifen;
- Figur 2: einen Schnitt durch die erfindungsgemäße Vornchtung mit einem eingespannten und konkav gekrümmten Probenstreifen;
- Figur 3: einen Schnitt durch einen konvex gekrümmten Probenstreifen.

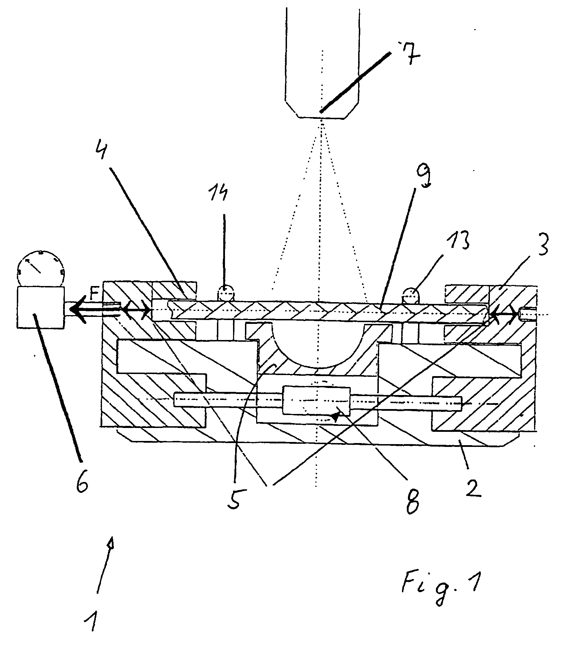

Die in Figur 1 schematisch dargestellte Vorrichtung 1 besteht im wesentlichen aus einer Trägerplatte 2, auf der zwei axial verschiebbare als Aufnahmen dienende Spannklötzen 3 und 4 und ein auswechselbarer Probenkörper 5 angeordnet sind.

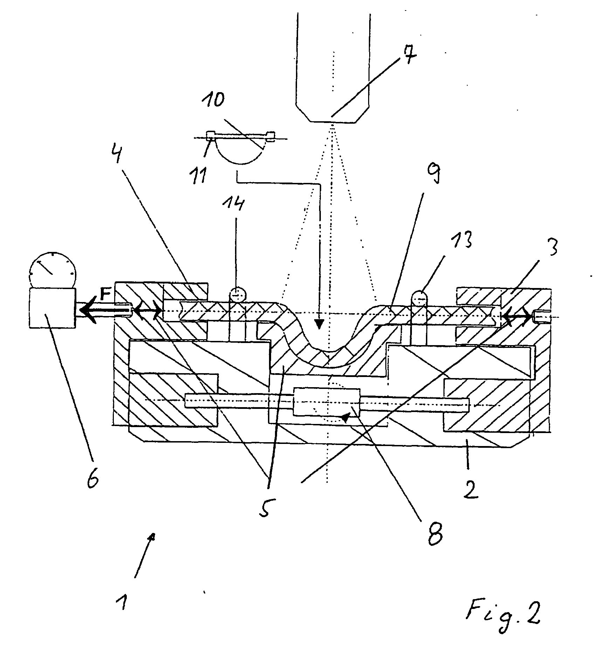

Der Probenkörper 5 gemäß Figur 2 weist eine rinnenförmige (konkave) Vertiefung auf.

Die Trägerplatte 2 ist auf einer Grundplatte (nicht dargestellt) angeordnet. Weiterhin sind auf dieser Grundplatte eine Kraft-/Wegmeßeinrichtung 6 sowie ein optischer Meßtaster 7 (Meßkamera mit Auswerteelektronik, PC und Bildschirmanzeige) vorgesehen. Die Kraft- und Wegmeßeinrichtung 6 greift an einem der Spannklötze 3 oder 4.

Die Verstellung der Spannklötze 3 und 4 erfolgt über eine Doppelspindel 8, von der Mitte ausgehend absolut gleichmäßig in beide Richtungen, entweder von Hand, über einen Schrittschaltmotor oder einen elektrischen Kugelspindelantrieb. Somit liegt das Meßfeld des Meßtasters 7 immer an der gleichen Stelle des Probenstreifens.

Zusätzlich sind Führungsrollen 13 und 14 mit seitlichen nicht näher dargestellten Fixierungen für den Probenstreifen vorgesehen.

Zum Ermitteln der Oberflächenqualität einer Haut wird zwischen beiden Spannklötzen 3 und 4 ein schmaler, länglicher Probenstreifen 9 (freie Länge 100 mm, Breite 20 mm), der aus der zu untersuchenden Haut ausgestanzt wurde, eingespannt. Die Einspannung ist so ausgestaltet, daß während der Ermittlung der Rauhtiefen und der Dehnung der Probenstreifen sicher gehalten und seitlich fixiert wird.

Zu Beginn der Messung wird der plane Probenstreifen in axialer Richtung durch Betätigen der Verschiebeeinrichtung mit einer Vorspannung von 1-2 N gedehnt. Dehnung und Dehnkraft können über die Kraft- und Wegmesseinrichtung 6 ermittelt bzw. abgelesen werden. Anschließend wird die Rauhtiefe des so gedehnten Probenstreifens mit Hilfe der optischen Meßeinrichtung 7 im Mittelbereich des Probenstreifens ermittelt. Hierbei wird der Probenstreifen so eingespannt, daß seine sichtbare Seite der sichtbaren Oberfläche am Fertigprodukt entspricht.

Als nächster Schritt werden die Spannklötze 3 und 4 so weit zueinander bewegt, daß sich die Vorspannung des Probenstreifens abgebaut und seine Unterseite in die rinnenförmige (konkave) Vertiefung in dem Probenkörper 5 (Figur 2) eingelegt werden kann. Die sichtbare Oberfläche wird somit gestaucht.

Mit Hilfe einer Fixiervorrichtung 10, die aus dünnem Federstahl besteht und mit Gewichten 11 versehen ist, wird der Probenstreifen 9 eng anliegend in die Vertiefung in dem Probenkörper 5 gedrückt und dort gehalten. Außerdem weist die Fixiervorrichtung 10 seitliche Halterungen für den Probenstreifen auf, damit sich dieser nicht verschieben kann.

Nunmehr wird erneut die Rauhtiefe im nun konkav gekrümmten Bereich gemessen. Beide Ergebnisse, die bei nicht ausgelenkten Probenstreifen und bei ausgelenkten Probenstreifen ermittelten Rauhtiefen, werden miteinander verglichen. Ist die prozentuale Änderung unter einem bestimmten Grenzwert, so eignet sich das Leder für die vorgesehene Weiterverarbeitung. Die Grenzwerte selbst werden in Testreihen ermittelt. Verhältniswerte von unter 100% bedeuten generell eine Eignung für eine Weiterverarbeitung. Ermittelt und verglichen werden bevorzugt die Mittenrauhwerte R_{a.}

Der Probenkörper 5 ist auswechselbar/verschiebbar angeordnet, um die Auslenkung des Probenstreifens entsprechend der zu messenden Lage ohne Umspannung der Lederprobe anpassen zu können. Dementsprechend existieren auch unterschiedliche Probenkörper.

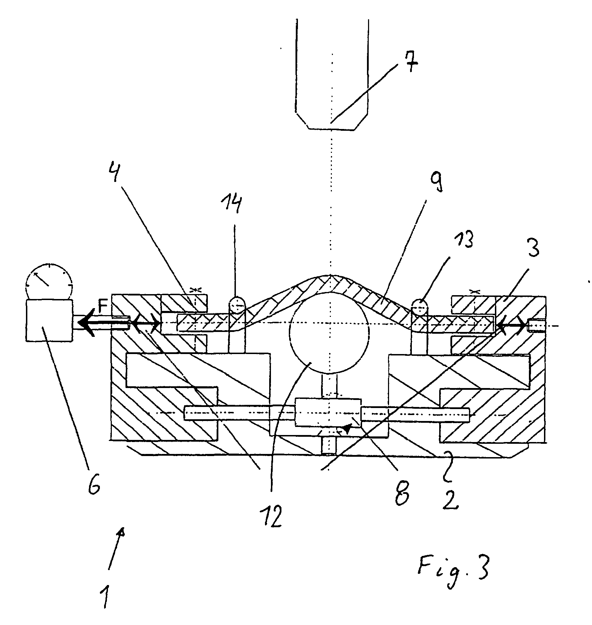

Um die Oberflächenstruktur bei einer Streckung der sichtbaren Oberfläche der Haut zu ermitteln, wird die Anordnung gemäß Figur 3 verwendet. Anstelle des Probenkörpers 5 wird eine Kugel 12 mit gleichem Radius wie der der rinnenförmigen Vertiefung verwendet.

Über die Axialverstellung der Spannklötze 3 und 4 wird eine definierte Eindrückkraft durch die Kugel auf den Probenstreifen 9 erzeugt. Die Narbung der Probe wird dabei flacher. Die Eindrückkraft bzw. die den Probenstreifen dehnende Kraft entspricht in ihrer Höhe der während der Weiterverarbeitung der Haut bzw. am späteren Fertigprodukt auftretenden Belastung im gewölbten Bereich. Übliche Kräfte liegen im Bereich von ca. 10 N .

Die Ermittlung der Rauhtiefe bei der Anordnung gemäß Figur 3 wird in Analogie zur Rauhtiefenermittlung gemäß Figur 2 durchgeführt und wiederum mit der an dem planen Probenstreifen 5 ermittelten ins Verhältnis gesetzt.

Mit der erfindungsgemäßen Vorrichtung und dem erfindungsgemäßen Meßverfahren ist eine objektive Beurteilung der Oberflächenstruktur von Häuten, insbesondere Lederhäuten, möglich. Die bisher angewandte subjektive Bewertung mit Hilfe von Grenzmustern kann entfallen. Aufgrund der exakten Messung von Narbentiefe bzw. Veränderung der Narbentiefe bei Stauchung oder Streckung lassen sich konkrete Schlüsse auf die Verarbeitbarkeit der Haut schließen. Ob die Messung bei Stauchung und Streckung stattfindet, hängt von der Kontur des Fertigproduktes ab. Ist die Haut am Fertigprodukt nur gestaucht oder gestreckt, braucht nur die entsprechende Messung durchgeführt zu werden. Damit ist eine frühzeitige Aussage über das Verhalten spezieller Häute bei entsprechenden Belastungen bereits in der Entwicklungsphase möglich, so daß unnötiger Aufwand und späterer Ausschuß entfallen kann.

Weiterhin lassen sich mit dem erfindungsgemäßen Meßverfahren den einzelnen Lederqualitäten Grenzwertbereiche zuweisen. Damit kann dann festgelegt werden, ob das Leder aufgrund der ermittelten Verhältniswerte unterhalb des ein oder anderen Grenzwertes liegt und damit für das ein oder andere Fertigprodukt geeignet ist.

Mit der erfindungsgemäßen Vorrichtung kann auch das statische und dynamische Dehnungsverhalten des Probenstreifens ermittelt werden. Hierzu wird er plan eingespannt und gedehnt. Der Kraftmesser zeigt die Dehnkraft an und über den Wegmesser kann der Längenzuwachs ermittelt werden. Daneben ist es auch möglich, den Kraftverlauf bei Belastung, nämlich das Elastizitätsverhalten des Probenstreifens am Kraftmesser, abzulesen.

## Patentansprüche

1. Verfahren zum Ermitteln der Verarbeitungsqualität von Häuten, insbesondere von Lederhäuten, bei dem ein Probenstreifen der Haut entnommen wird und dessen Oberfläche untersucht wird,
**dadurch gekennzeichnet, dass** als Maß der Verarbeitungsqualität die Rauhtiefe des Probenstreifens bei einer definierten Vorspannung in Längsrichtung planliegend ermittelt wird und dass dann der Probenstreifen gekrümmt wird, wobei dann erneut die Rauhtiefe im Krümmungsbereich ermittelt und mit der Rauhtiefe im ungekrümmten Zustand verglichen wird.

2. Verfahren nach Anspruch 1,
**dadurch gekennzeichnet, dass** der Probenstreifen zuerst konkav und dann konvex gekrümmt wird, wobei nach jedem Krümmungsvorgang die Rauhtiefe gemessen wird.

3. Verfahren nach Anspruch 1 oder 2,
**dadurch gekennzeichnet, dass** der Probenstreifen derart gekrümmt wird, dass die Mittellage des Probenstreifens im gleichen Messbereich liegt wie bei planliegender Messung.

4. Verfahren nach einem der vorangegangenen Ansprüche,
**dadurch gekennzeichnet, dass** der Probenstreifen während der Krümmung mit einer Dehnungskraft belastet wird, deren Höhe der bei der späteren Weiterverarbeitung auftretenden Kraft entspricht.

5. Verfahren nach einem der vorangegangenen Ansprüche,
**dadurch gekennzeichnet, dass** die Rauhtiefe optisch oder mechanisch ermittelt wird.

6. Vorrichtung zur Ermittlung der Oberflächenstruktur von Häuten, insbesondere von Lederhäuten mit Hilfe eines Probenstreifens der Haut,
wobie zwei Aufnahmen (3, 4) vorgesehen sind, die auf einer Trägerplatte (2) zum Einspannen des Probestreifens (9) in Längsrichtung angeordnet sind, von denen mindestens eine in Längsrichtung des Probestreifens (9) verstellbar ist, eine den Probestreifen (9) stauchende oder streckende Einrichtung und eine die Rauhtiefe erfassende Messvorrichtung (7) zwischen den beiden Aufnahmen vorgesehen ist und an einer längsverschiebbaren Aufnahmen (3,4) eine dehnkrafterzeugende und messende Einrichtung (6) angreift.

7. Vorrichtung nach Anspruch 6,
**dadurch gekennzeichnet, dass** jede Aufnahme (3, 4) axial verschiebbar und feststellbar angeordnet ist.

8. Vorrichtung nach Anspruch 6 oder 7,
**dadurch gekennzeichnet, dass** an einer Aufnahme (3 oder 4) oder an der Trägerplatte (2) eine Wegmesseinrichtung vorgesehen ist.

9. Vorrichtung nach Anspruch 8,
**dadurch gekennzeichnet, dass** sie eine Einstellschraube (8) als aufweist längskrafterzeugende Einrichtung, die beide Aufnahmen (3, 4) in Längsrichtung des Probenstreifens (9) gleichzeitig verschiebt.

10. Vorrichtung nach einem der Ansprüche 6 bis 9,
**dadurch gekennzeichnet, dass** die die Stauchung der Oberfläche des Probenstreifens (9) erzeugende Einrichtung aus einem Probenkörper (5) mit einer rinnenförmigen Ausnehmung besteht und dass die Messeinrichtung (7) auf der gestauchten Oberseite des Probenstreifens (9) misst.

11. Vorrichtung nach einem der vorangegangenen Ansprüche 6 bis 10,
**dadurch gekennzeichnet, dass** die die Streckung erzeugende Einrichtung ein kugelförmiger Probenkörper (12) ist, der unterhalb des Probenstreifens (9) angeordnet ist, und dass die Messeinrichtung (7) auf der gestreckten Oberseite des Probenstreifens (9) misst.

12. Vorrichtung nach einem der vorangegangenen Ansprüche 10 order 11,
**dadurch gekennzeichnet, dass** der jeweilige Probenkörper (5, 12) auswechselbar angeordnet sind.

13. Vorrichtung nach Anspruch 10,
**dadurch gekennzeichnet, dass** ein Andrückblech (10) vorgesehen ist, das den Probenstreifen (9) in die rinnenförmige Aufnahme des Probenkörpers (5) drückt und dort hält.

14. Vorrichtung nach Anspruch 13,
**dadurch gekennzeichnet, dass** das Andrückblech (10) ein Federblech ist, das mit Zusatzgewichten (11) ausgestattet ist.

15. Vorrichtung nach einem der vorangegangenen Ansprüche 6 bis 14,
**dadurch gekennzeichnet, dass** als Messvorrichtung (7) ein mechanischer Messtaster verwendet wird.

16. Vorrichtung nach einem der Ansprüche 6 bis 14,
**dadurch gekennzeichnet, dass** als Messvorrichtung (7) ein optisches Messgerät verwendet wird.

## Claims

1. A method of determining the processing quality of skins, especially leather skins, wherein a test strip is taken from the skin and its surface is investigated,
**characterised in that** the measure of the processing quality is the surface roughness of the test strip under a defined prestress in the longitudinal direction and determined when flat, after which the test strip is bent and the surface roughness is again measured in the bent region and compared with the surface roughness in the unbent state.

2. A method according to claim 1,
**characterised in that** the test strip is bent first concavely and then convexly and the surface roughness is measured after each bending operation.

3. A method according to claim 1 or claim 2,
**characterised in that** the test strip is bent so that the centre position of the test strip lies in the same measuring range as when measured when flat.

4. A method according to any of the preceding claims,
**characterised in that** while being bent, the test strip is loaded with a stretching force equal in value to the force occurring during subsequent further processing.

5. A method according to any of the preceding claims,
**characterised in that** the surface roughness is measured optically or mechanically.

6. A device for determining the surface structure of skins, especially leather skins, by using a test strip of the skin, wherein two holders (3, 4) are provided and disposed on a supporting plate (2) for clamping the test strip (9) in the longitudinal direction, at least one holder being adjustable in the longitudinal direction of the test strip (9), a device for compressing or stretching the test strip (9) and a device (7) for measuring the surface roughness are provided between the two holders and a device (6) for generating and measuring a stretching force engages one of the longitudinally movable holders (3, 4).

7. A device according to claim 6,
**characterised in that** each holder (3, 4) is axially movable and lockable.

8. A device according to claim 6 or claim 7,
**characterised in that** a position-measuring device is provided on a holder (3 or 4) or on the supporting plate (2).

9. A device according to claim 8,
**characterised in that** it comprises a longitudinal force-generating device in the form of an adjusting screw (8), which moves the two holders (3, 4) simultaneously in the longitudinal direction of the test strip (9).

10. A device according to any of claims 6 to 9,
**characterised in that** the device for compressing the surface of the test strip (9) comprises a test member (5) with a channel-like recess, and the measuring device (7) makes measurements on the compressed top of the test strip.

11. A device according to any of the preceding claims 6 to 10,
**characterised in that** the stretching device is a spherical test member (12) which is disposed under the test strip (9), and the measuring device (7) makes measurements on the stretched surface of the test strip (9).

12. A device according to any of the preceding claims 10 or 11,
**characterised in that** each test member (5, 12) is interchangeable.

13. A device according to claim 10,
**characterised in that** a pressure plate (10) is provided and presses the test strip (9) into the channel-like holder in the test member (5) and holds it there.

14. A device according to claim 13,
**characterised in that** the pressure plate (10) is a spring plate equipped with additional weights (11).

15. A device according to any of the preceding claims 6 to 14,
**characterised in that** the measuring device (7) is a mechanical measuring scanner.

16. A device according to any of claims 6 to 14,
**characterised in that** the measuring device (7) is an optical measuring instrument.

## Revendications

1. Procédé de détermination de la qualité du traitement des peaux, en particulier des peaux en cuir, selon lequel on prélève une bande d'échantillon de peau et on analyse sa surface,
**caractérisé en ce que**
pour mesurer la qualité du traitement, on détermine à plat, dans le sens de la longueur la profondeur de rugosité de la bande d'échantillon sous une précontrainte définie, et
on recourbe ensuite la bande d'échantillon, la profondeur de rugosité étant à nouveau déterminée dans la zone de courbure et comparée à la profondeur de rugosité à l'état non courbé.

2. Procédé selon la revendication 1,
**caractérisé en ce que**
la bande d'échantillon est tout d'abord recourbée de manière concave, puis de manière convexe, la profondeur de rugosité étant mesurée après chaque processus de courbure.

3. Procédé selon la revendication 1 ou 2,
**caractérisé en ce que**
la bande d'échantillon est recourbée de telle sorte que la position centrale de la bande d'échantillon soit dans la même étendue de mesure que lors de la mesure à plat.

4. Procédé selon l'une quelconque des revendications précédentes,
**caractérisé en ce que**
la bande d'échantillon est soumise, pendant la courbure, à une force d'allongement dont la grandeur correspond à la force produite pendant la transformation ultérieure.

5. Procédé selon l'une quelconque des revendications précédentes,
**caractérisé en ce que**
la profondeur de rugosité est déterminée de manière optique ou mécanique.

6. Dispositif de détermination de la structure superficielle des peaux, en particulier des peaux en cuir, à l'aide d'une bande d'échantillon de peau,
dans lequel
deux réceptions (3, 4) sont prévues sur une plaque de support (2) servant à tendre la bande d'échantillon (9) dans le sens de la longueur, dont au moins une réception est réglable dans le sens de la longueur de la bande d'échantillon, avec un système serrant ou étirant la bande d'échantillon (9) et un système de mesure (7) enregistrant la profondeur de rugosité étant prévus entre les deux réceptions, et un système (6) de production de la force d'allongement et de mesure empiétant sur l'une des réceptions (3, 4) mobiles dans le sens de la longueur.

7. Dispositif selon la revendication 6,
**caractérisé en ce que**
chaque réception (3, 4) est mobile axialement et peut être fixée.

8. Dispositif selon la revendication 6 ou 7,
**caractérisé en ce qu'**
un système de mesure de la distance est prévu au niveau d'une réception (3 ou 4) ou au niveau de la plaque de support (2).

9. Dispositif selon la revendication 8,
**caractérisé en ce qu'**
il présente une vis de réglage (8) servant de système de production d'une force longitudinale qui déplace simultanément les deux réceptions (3, 4) dans le sens de la longueur de la bande d'échantillon (9).

10. Dispositif selon l'une quelconque des revendications 6 à 9,
**caractérisé en ce que**
le système produisant le serrage de la surface de la bande d'échantillon (9) est composé d'un corps d'échantillonnage (5) comprenant un évidement en forme de rainure et le système de mesure (7) mesure la face supérieure serrée de la bande d'échantillon (9).

11. Dispositif selon l'une quelconque des revendications précédentes 6 à 10,
**caractérisé en ce que**
le système produisant l'étirement est un corps d'échantillonnage sphérique (12) qui est disposé en dessous de la bande d'échantillon (9), et le système de mesure (7) mesure la surface étirée de la bande d'échantillon (9).

12. Dispositif selon l'une quelconque des revendications précédentes 10 ou 11,
**caractérisé en ce que**
les corps d'échantillonnage (5, 12) respectifs sont disposés de manière à être amovibles.

13. Dispositif selon la revendication 10.
**caractérisé en ce qu'**
une tôle d'appui (10) est prévue pour pousser la bande d'échantillon (9) dans la réception en forme de rainure du corps d'échantillonnage (5) et l'y maintenir.

14. Dispositif selon la revendication 13,
**caractérisé en ce que**
la tôle d'appui (10) est une tôle à ressort équipée de poids supplémentaires (11).

15. Dispositif selon l'une quelconque des revendications précédentes 6 à 14,
**caractérisé en ce que**
pour le système de mesure (7), on utilise un poste de mesure mécanique.

16. Dispositif selon l'une quelconque des revendications 6 à 14,
**caractérisé en ce que**
pour le système de mesure (7), on utilise un appareil de mesure optique.
